# EUROPEAN PATENT SPECIFICATION

(11) **EP 0 937 040 B1**
(45) Date of publication and mention of the grant of the patent: **21.07.2004**
(21) Application number: 97950112.9
(22) Date of filing: 05.11.1997
(51) Int. Cl.: C07D 207/00, C07D 401/00, C07K 5/00

(54) **N-SUBSTITUTED 2-CYANOPYRROLIDINES**
N-SUBSTITUIERTE 2-CYANOPYRROLIDINE
2-CYANOPYRROLIDINES A SUBSTITUTION N

(30) Priority: 07.11.1996 US 746295
(43) Date of publication of application: 25.08.1999
(73) Proprietor: Novartis AG, 4056 Basel (CH); Novartis Pharma GmbH, 1230 Wien (AT)
(72) Inventor: VILLHAUER, Edwin, Bernard, Morristown, NJ 07960 (US)
(74) Representative: Grubb, Philip William
(86) International application number: PCT/EP1997/006125
(87) International publication number: WO 1998/019998

(56) References cited:
- EP-A- 0 115 639
- EP-A- 0 132 580
- EP-A- 0 172 458
- WO-A-90/12005

## Description

### Field

The present invention relates to N-substituted 2-cyanopyrrolidines. More particularly, it provides novel N-glycyl-2-cyanopyrrolidine derivatives.

### Background

Dipeptidyl peptidase-IV **(DPP-IV)** is a serine protease which cleaves N-terminal dipeptides from a peptide chain containing, preferably, a proline residue in the penultimate position. Although the biological role of DPP-IV in mammalian systems has not been completely established, it is believed to play an important role in neuropeptide metabolism, T-cell activation, attachment of cancer cells to the endothelium and the entry of HIV into lymphoid cells. DPP-IV is responsible for inactivating glucagon-like peptide-1 **(GLP-1).** More particularly, DPP-IV cleaves the amino-terminal His-Ala dipeptide of GLP-1, generating a GLP-1 receptor antagonist, and thereby shortens the physiological response to GLP-1. Since the half-life for DPP-IV cleavage is much shorter than the half-life for removal of GLP-1 from circulation, a significant increase in GLP-1 bioactivity (5- to 10-fold) is anticipated from DPP-IV inhibition. Since GLP-1 is a major stimulator of pancreatic insulin secretion and has direct beneficial effects on glucose disposal, DPP-IV inhibition appears to represent an attractive approach for treating non-insulin-dependent diabetes mellitus **(NIDDM).**

Although a number of DPP-IV inhibitors have been described, all have limitations relating to potency, stability or toxicity. Accordingly, a great need exists for novel DPP-IV inhibitors which are useful in treating conditions mediated by DPP-IV inhibition and which do not suffer from the above-mentioned limitations.

### Summary of the invention

The invention provides novel N-(N'-substituted glycyl)-2-cyanopyrrolidines which are effective as DPP-IV inhibitors in treating conditions mediated by DPP-IV. It also concerns corresponding pharmaceutical compositions, a process for their preparation, a method of inhibiting DPP-IV comprising administering to a patient in need of such treatment a therapeutically effective amount thereof, the compounds for use as a pharmaceutical, and their use in a process for the preparation of a medicament for treating a condition mediated by DPP-IV.

### Detailed description

The invention concerns N-(N'-substituted glycyl)-2-cyanopyrrolidines of formula I: 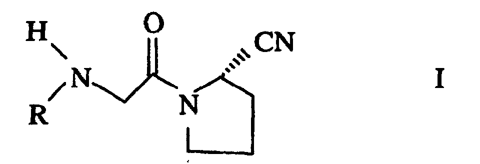 wherein **R** is:
a) **R**_{**1**}**R**_{**1a**}**N(CH**_{**2**}**)**_{**m**}**-** wherein
   - R₁: is a pyridinyl or pyrimidinyl moiety optionally mono- or independently disubstituted with (C₁₋₄)alkyl, (C₁₋₄)alkoxy, halogen, trifluoromethyl, cyano or nitro; or phenyl optionally mono- or independently disubstituted with (C₁₋₄)alkyl, (C₁₋₄)alkoxy or halogen;
   - R₁ₐ: is hydrogen or (C₁₋₈)alkyl; and
   - m: is 2 or 3;
b) **(C**_{**3-12**}**)cycloalkyl** optionally monosubstituted in the 1-position with (C₁₋₃)hydroxyalkyl;
c) **R**_{**2**}**(CH**_{**2**}**)**_{**n**}**-** wherein either
   - R₂: is phenyl optionally mono- or independently di- or independently trisubstituted with (C₁₋₄)alkyl, (C₁₋₄)alkoxy, halogen or phenylthio optionally monosubstituted in the phenyl ring with hydroxymethyl; or is (C₁₋₈)alkyl; a [3.1.1]bicyclic carbocyclic moiety optionally mono- or plurisubstituted with (C₁₋₈)alkyl;
   a pyridinyl or naphthyl moiety optionally mono- or independently disubstituted with (C₁₋₄)alkyl, (C₁₋₄)alkoxy or halogen; cyclohexene; or adamantyl; and
   - n: is 1 to 3; or
   - R₂: is phenoxy optionally mono- or independently disubstituted with (C₁₋₄)alkyl, (C₁₋₄)alkoxy or halogen; and
   - n: is 2 or 3;
d) **(R**_{**3**}**)**_{**2**}**CH(CH**_{**2**}**)**_{**2**}**-** wherein each R₃ independently is phenyl optionally mono- or independently disubstituted with (C₁₋₄)alkyl, (C₁₋₄)alkoxy or halogen;
e) **R**_{**4**}**(CH**_{**2**}**)**_{**p**}**-** wherein R₄ is 2-oxopyrrolidinyl or (C₂₋₄)alkoxy, and p is 2 to 4;
f) **isopropyl** optionally monosubstituted in 1-position with (C₁₋₃)hydroxyalkyl;
g) **R**_{**5**} wherein R₅ is: indanyl; a pyrrolidinyl or piperidinyl moiety optionally substituted with benzyl; a [2.2.1]- or [3.1.1]bicyclic carbocyclic moiety optionally mono- or plurisubstituted with (C₁₋₈)alkyl; adamantyl; or (C₁₋₈)alkyl optionally mono- or independently plurisubstituted with hydroxy, hydroxymethyl or phenyl optionally mono- or independently disubstituted with (C₁₋₄)alkyl, (C₁₋₄)alkoxy or halogen;
in free form or in acid addition salt form.
Compounds having different structures were described in the patent applications EP 0 123 580, EP 0 115 639, EP 0 172 458 and WO 90/12005. However, none of these documents, disclosed the use of such compounds as DPP-IV inhibitors.

The compounds of formula I can exist in free form or in acid addition salt form. Salt forms may be recovered from the free form in known manner and vice-versa. Acid addition salts may e.g. be those of pharmaceutically acceptable organic or inorganic acids. Although the preferred acid addition salts are the hydrochlorides, salts of methanesulfonic, sulfuric, phosphoric, citric, lactic and acetic acid may also be utilized.

The compounds of the invention may exist in the form of optically active isomers or diastereoisomers and can be separated and recovered by conventional techniques, such as chromatography.

"Alkyl" and "alkoxy" are either straight or branched chain, of which examples of the latter are isopropyl and tert-butyl.

R preferably is a), b) or e) as defined above. R₁ preferably is a pyridinyl or pyrimidinyl moiety optionally substituted as defined above. R₁ₐ preferably is hydrogen. R₂ preferably is phenyl optionally substituted as defined above. R₃ preferably is unsubstituted phenyl. R₄ preferably is alkoxy as defined above. R₅ preferably is optionally substituted alkyl as defined above. m preferably is 2. n preferably is 1 or 2, especially 2. p preferably is 2 or 3, especially 3.

Pyridinyl preferably is pyridin-2-yl; it preferably is unsubstituted or monosubstituted, preferably in 5-position. Pyrimidinyl preferably is pyrimidin-2-yl. It preferably is unsubstituted or monosubstituted, preferably in 4-position. Preferred as substitutents for pyridinyl and pyrimidinyl are halogen, cyano and nitro, especially chlorine.

When it is substituted, phenyl preferably is monosubstituted; it preferably is substituted with halogen, preferably chlorine, or methoxy. It preferably is substituted in 2-, 4- and/or 5-position, especially in 4-position.

(C₃₋₁₂)cycloalkyl preferably is cyclopentyl or cyclohexyl. When it is substituted, it preferably is substituted with hydroxymethyl. (C₁₋₄)alkoxy preferably is of 1 or 2 carbon atoms, it especially is methoxy. (C₂₋₄)alkoxy preferably is of 3 carbon atoms, it especially is isopropoxy. Halogen is fluorine, chlorine, bromine or iodine, preferably fluorine, chlorine or bromine, especially chlorine. (C₁₋₈)alkyl preferably is of 1 to 6, preferably 1 to 4 or 3 to 5, especially of 2 or 3 carbon atoms, or methyl. (C₁₋₄) alkyl preferably is methyl or ethyl, especially methyl. (C₁₋₃)hydroxyalkyl preferably is hydroxymethyl.

A [3.1. 1]bicyclic carbocyclic moiety optionally substituted as defined above preferably is bicyclo[3.1.1]hept-2-yl optionally disubstituted in 6-position with methyl, or bicyclo[3.1.1]hept-3-yl optionally trisubstituted with one methyl in 2-position and two methyl groups in 6-position. A [2.2.1]bicyclic carbocyclic moiety optionally substituted as defined above preferably is bicyclo[2.2.1]hept-2-yl.

Naphthyl preferably is 1-naphthyl. Cyclohexene preferably is cyclohex-1-en-1-yl. Adamantyl preferably is 1- or 2-adamantyl.

A pyrrolidinyl or piperidinyl moiety optionally substituted as defined above preferably is pyrrolidin-3-yl or piperidin-4-yl. When it is substituted it preferably is N-substituted.

A preferred group of compounds of the invention is the compounds of formula I wherein **R** is **R' (compounds la),** whereby R' is:
- R₁'NH(CH₂)₂- wherein R₁' is pyridinyl optionally mono- or independently disubstituted with halogen, trifluoromethyl, cyano or nitro; or unsubstituted pyrimidinyl;
- (C₃₋₇)cycloalkyl optionally monosubstituted in 1-position with (C₁₋₃)hydroxyalkyl;
- R₄'(CH₂)₃- wherein R₄' is (C₂₋₄)alkoxy; or
- R₅, wherein R₅ is as defined above;
in free form or in acid addition salt form.

More preferred compounds of the invention are those compounds of formula I wherein **R** is **R" (compounds Ib),** whereby R" is:
- R₁"NH(CH₂)₂- wherein R₁" is pyridinyl mono- or independently disubstituted with halogen, trifluoromethyl, cyano or nitro;
- (C₄₋₆)cycloalkyl monosubstituted in 1-position with (C₁₋₃)hydroxyalkyl;
- R₄'(CH₂)₃- wherein R₄' is as defined above; or
- R₅' wherein R₅' is a [2.2.1]- or [3.1.1]bicyclic carbocyclic moiety optionally mono- or plurisubstituted with (C₁₋₈)alkyl; or adamantyl;
in free form or in acid addition salt form.

Even more preferred compounds of the invention are the compounds of formula I wherein **R** is **R"' (compounds Ic),** whereby **R"'** is:
- R₁"NH(CH₂)₂- wherein R₁" is as defined above;
- (C₄₋₆)cycloalkyl monosubstituted in 1-position with hydroxymethyl;
- R₄'(CH₂)₃- wherein R₄' is as defined above; or
- R₅" wherein R₅" is adamantyl;
in free form or in acid addition salt form.

A further group of compounds of the invention is **compounds Ip**, wherein R is **R**^{**p**}, which is:
a) R₁^{p}NH(CH₂)₂- wherein R₁^{p} is a pyridinyl or pyrimidinyl moiety optionally mono- or independently disubstituted with halogen, trifluoromethyl, cyano or nitro;
b) (C₃₋₇)cycloalkyl optionally monosubstituted in 1-position with (C₁₋₃)hydroxyalkyl;
c) R₂^{p}(CH₂)₂- wherein R₂^{p} is phenyl optionally mono- or independently di- or independently trisubstituted with halogen or (C₁₋₃)alkoxy;
d) (R₃^{p})₂CH(CH₂)₂- wherein each R₃^{p} independently is phenyl optionally monosubstituted with halogen or (C₁₋₃)alkoxy;
e) R₄(CH₂)₃- wherein R₄ is as defined above; or
f) isopropyl optionally monosubstituted in 1-position with (C₁₋₃)hydroxyalkyl;
in free form or in pharmaceutically acceptable acid addition salt form.

A further group of compounds of the invention is **compounds Is,** wherein R is **R**^{**s**}**,** which is:
a) R₁^{s}R₁ₐ^{s}(CH₂)ₘₛ- wherein R₁^{s} is pyridinyl optionally mono- or independently disubstituted with chlorine, trifluoromethyl, cyano or nitro; pyrimidinyl optionally monosubstituted with chlorine or trifluoromethyl; or phenyl;
   R₁ₐ^{s} is hydrogen or methyl; and
   ms is 2 or 3;
b) (C₃₋₁₂)cycloalkyl optionally monosubstituted in 1-position with hydroxymethyl;
c) R₂^{s}(CH₂)ₙₛ- wherein either
   - R₂^{s}: is phenyl optionally mono- or independently di- or independently trisubstituted with halogen, alkoxy of 1 or 2 carbon atoms or phenylthio monosubstituted in the phenyl ring with hydroxymethyl; (C₁₋₆)alkyl; 6,6-dimethylbicyclo[3.1.1]hept-2-yl; pyridinyl; naphthyl; cyclohexene; or adamantyl; and ns is 1 to 3; or
   - R₂^{s}: is phenoxy; and ns is 2;
d) (3,3-diphenyl)propyl;
e) R₄^{s}(CH₂)ₚₛ wherein R₄^{s} is 2-oxopyrrolidin-1-yl or isopropoxy and
   ps is 2 or 3;
f) isopropyl optionally monosubstituted in 1-position with hydroxymethyl;
g) R₅^{s} wherein R₅^{s} is: indanyl; a pyrrolidinyl or piperidinyl moiety optionally N-substituted with benzyl; bicyclo[2.2.1]hept-2-yl; 2,6,6-trimethylbicyclo[3.1.1]hept-3-yl; adamantyl; or (C₁₋₈)alkyl optionally mono- or independently disubstituted with hydroxy, hydroxymethyl or phenyl;
in free form or in acid addition salt form.

The compounds of the invention may be prepared by a process which comprises coupling a reactive (2-cyanopyrrolidino)carbonylmethylene compound with an appropriate substituted amine; more particularly, for the preparation of the compounds of formula I it comprises reacting a compound of formula II 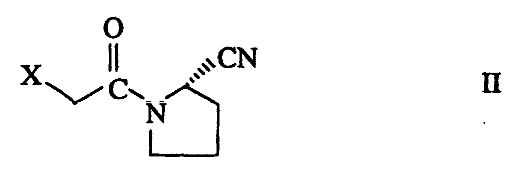 wherein X is a halogen such as bromine, chlorine or iodine,
with a compound of formula III

NH₂R III

wherein R is as defined above,
and recovering the resultant compound of formula I in free form or in acid addition salt form.

The process of the invention may be effected in conventional manner.

The compound of formula II is preferably reacted with at least 3 equivalents of a primary amine of formula III. The reaction is conveniently conducted in the presence of an inert, organic solvent, preferably a cyclic ether such as tetrahydrofuran. The temperature preferably is of from about 0° to about 35°C, preferably between about 0° and about 25°C.

The compounds of the invention may be isolated from the reaction mixture and purified in conventional manner, e.g. by chromatography.

The starting materials may also be prepared in conventional manner.

The compounds of formula II may e.g. be prepared by the following two-step reaction. scheme:

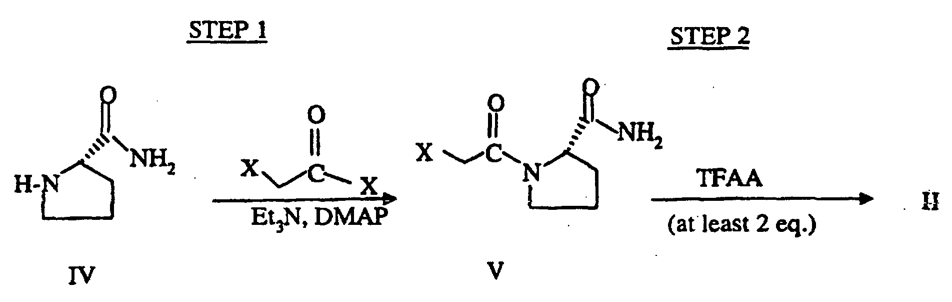

Step 1 involves the reaction of the pyrrolidine of formula IV with a slight molar excess of a haloacetylhalide such as bromoacetylbromide or chloroacetylchloride and triethylamine and a catalytic amount of dimethylaminopyridine (DMAP). The reaction conveniently is conducted in the presence of an inert, organic solvent, preferably a chlorinated, aliphatic hydrocarbon such as methylene chloride, at a temperature of from about 0° to about 25°C, preferably at a temperature between about 0° and about 15°C.

Step 2 concerns the dehydration of the compound of formula V, prepared in Step 1, with at least 2 equivalents of trifluoroacetic anhydride (TFAA). The dehydration preferably is conducted in the presence of an inert, organic solvent such as tetrahydrofuran or a chlorinated, aliphatic hydrocarbon such as methylene chloride, at a temperature of from about 0° to about 25°C, preferably at a temperature between about 0° and about 15°C.

Insofar as its preparation is not particularly described herein, a compound used as starting material is known or may be prepared from known compounds in known manner or analogously to known methods or analogously to methods described in the Examgles.

The following Examples illustrate the invention. All temperatures are in degrees Celsius.

### Example 1: 1-[2-[(5-Chloropyridin-2-yl)amino]ethylamino]acetyl-2-cyano-(S)-pyrrolidine

To a 500 ml flask is added 16.6 g of **2-[(5-chloropyridin-2-yl)amino]ethylamine** and 100 ml of tetrahydrofuran and the mixture is cooled in an ice bath. To the cooled mixture is added 7.0 g of **(2-cyanopyrrolidino)carbonylmethylene-(S)-bromide** dissolved in 30 ml of tetrahydrofuran. The resultant mixture is stirred for 2 hours at 0°, the solvent is removed by rotovaping and the mixture is partitioned between ethyl acetate and water. The product is then extracted into the ethyl acetate layer and the aqueous layer is then washed twice with ethyl acetate. The combined organic layers are washed successively with water and brine, dried over sodium sulfate and concentrated to obtain the desired free base compound in crude form. The crude form is then purified on silica gel employing a mixture of 5% methanol in methylene chloride as the eluent to yield the **title compound in free base form** as a light brown oil.

After dissolving the **free base** in 30 ml of dry tetrahydrofuran, **hydrogen chloride** gas is bubbled into the solution for five seconds. The off-white precipitate that forms is filtered, washed with dry tetrahydrofuran and the solvent is removed by high vacuum pumping to obtain the **title compound in dihydrochloride acid addition salt form** (off-white solid; m.p. 265°-267°; NMR: see * at bottom of Table hereunder).

The starting material is obtained as follows:
a) 22.37 g of **(S)-2-carbamoylpyrrolidine,** 30.1 ml of triethylamine and 30.0 mg of dimethylaminopyridine **(DMAP)** are dissolved in 200 ml of methylene chloride and the solution is then added, dropwise, to an ice-cold solution of 18.8 ml of **bromoacetylbromide** in 192 ml of methylene chloride, over a period of 60 minutes under a calcium sulfate drying tube. The resultant solution is stirred for 2 hours at ice-water temperature under a calcium sulfate drying tube, then poured into 3.5 liters of ethyl acetate. The resultant precipitate is filtered, washed with ethyl acetate, and the filtrate is concentrated to obtain **(2-carbamoylpyrrolidino)-carbonylmethylene-(S)-bromide** (hard yellow taffy).
b) 50.0 g of the **bromide compound** prepared in a) above is dissolved in 300 ml of methylene chloride and the solution is cooled in an ice water bath under a calcium sulfate drying tube.
The cooled solution is then poured into 60.2 ml of **trifluoroacetic anhydride** over a 2 minute period, the resultant solution is stirred at ice-water temperature under a calcium sulfate drying tube for 4 hours, and partitioned between methylene chloride and saturated aqueous sodium bicarbonate. The product is extracted into the methylene chloride layer and the aqueous layer is washed twice with methylene chloride. The combined organic layers are washed successively with water and brine and then dried over sodium sulfate. The solution is filtered and the solvent is removed by rotovaping and high vacuum pumping to obtain **(2-cyanopyrrolidino)carbonylmethylene-(S)-bromide** (dark yellow solid).

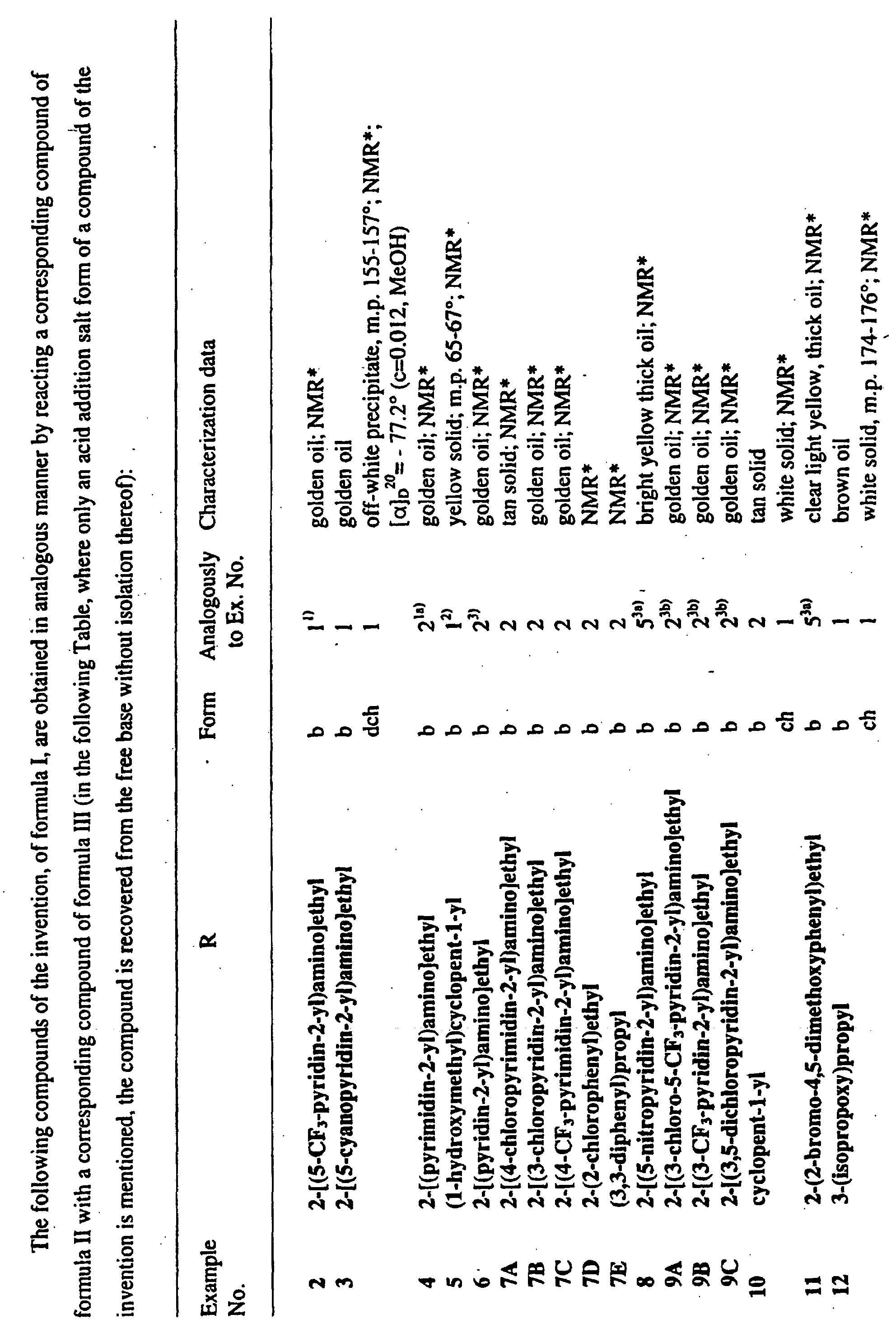

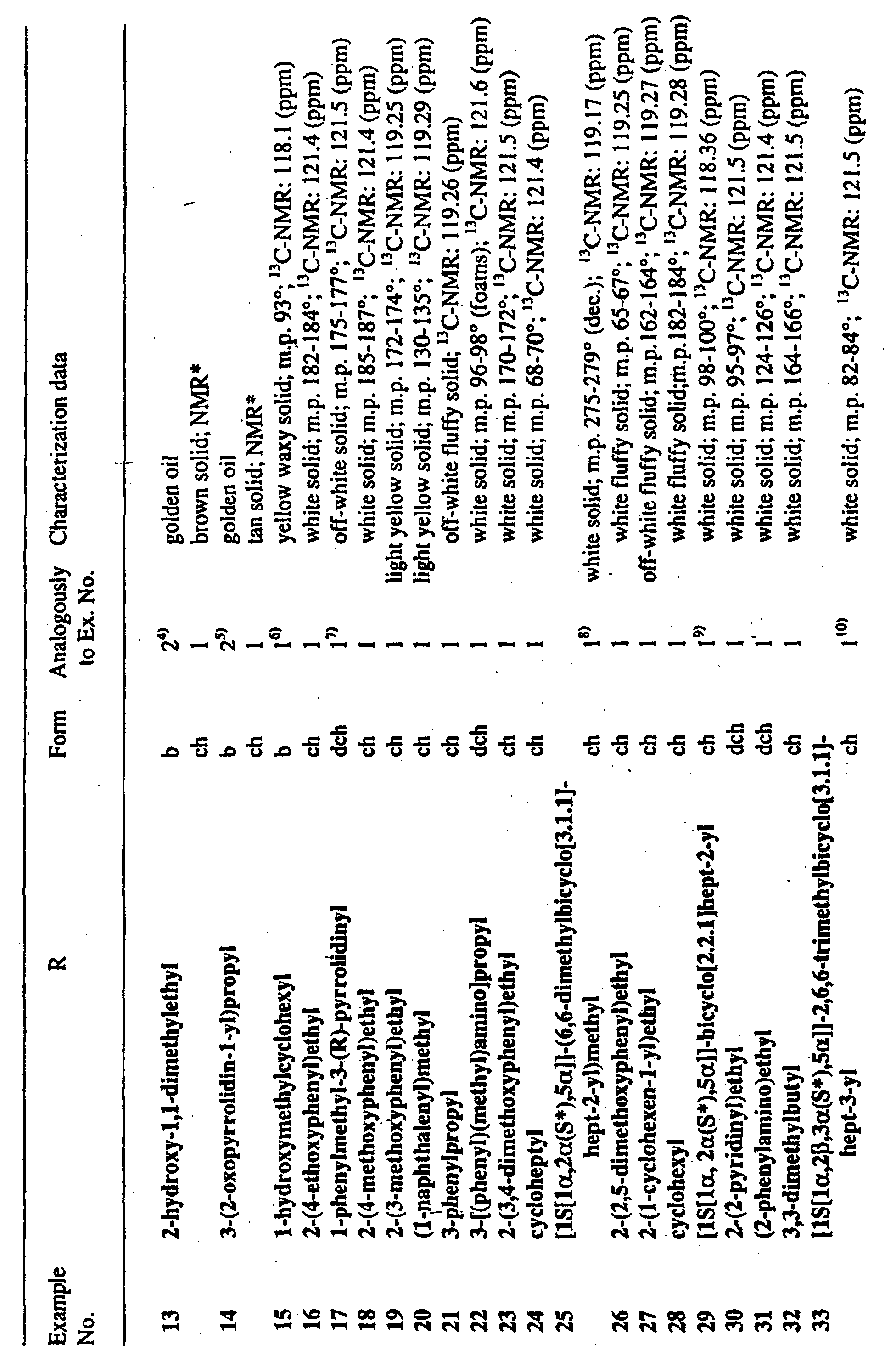

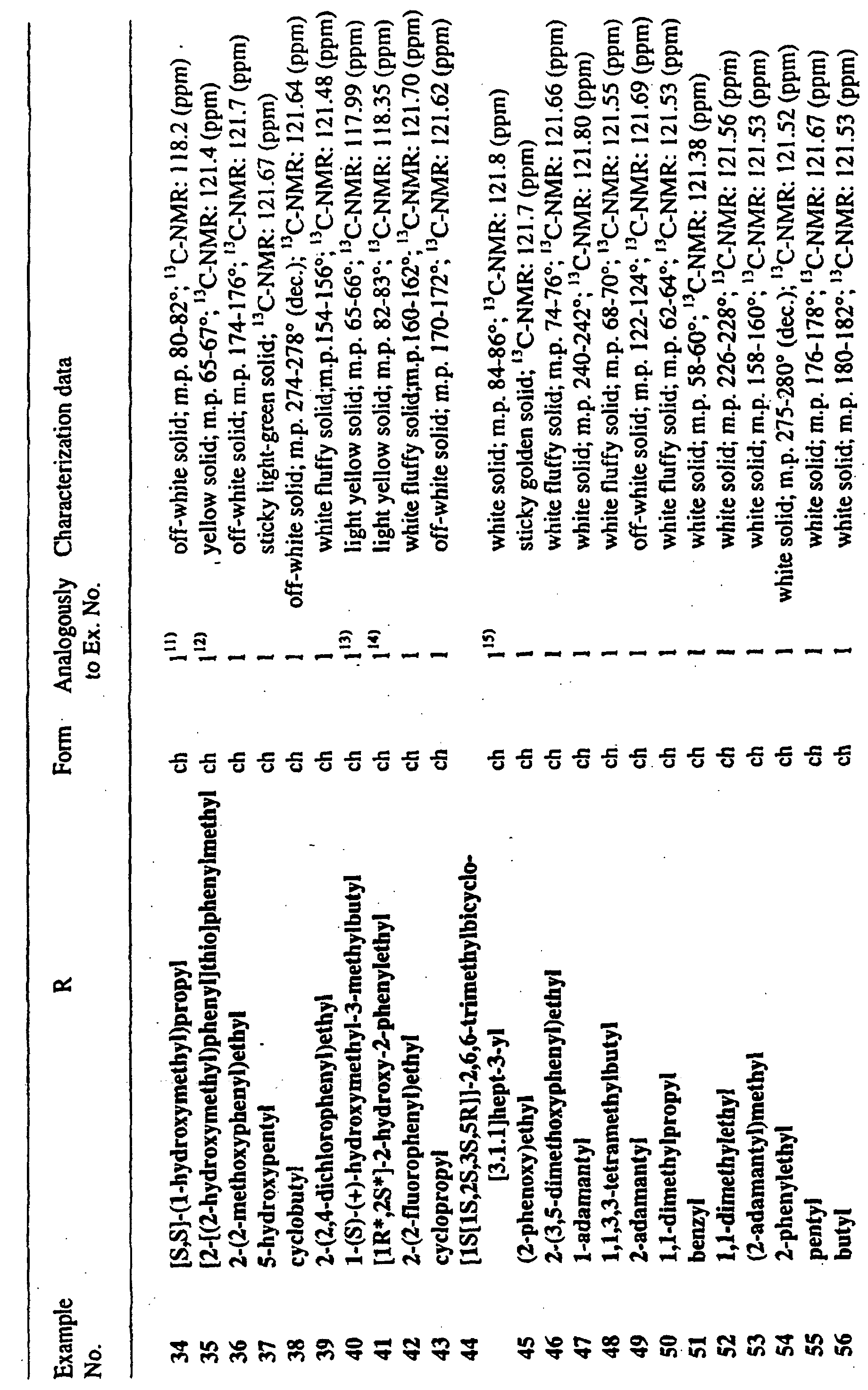

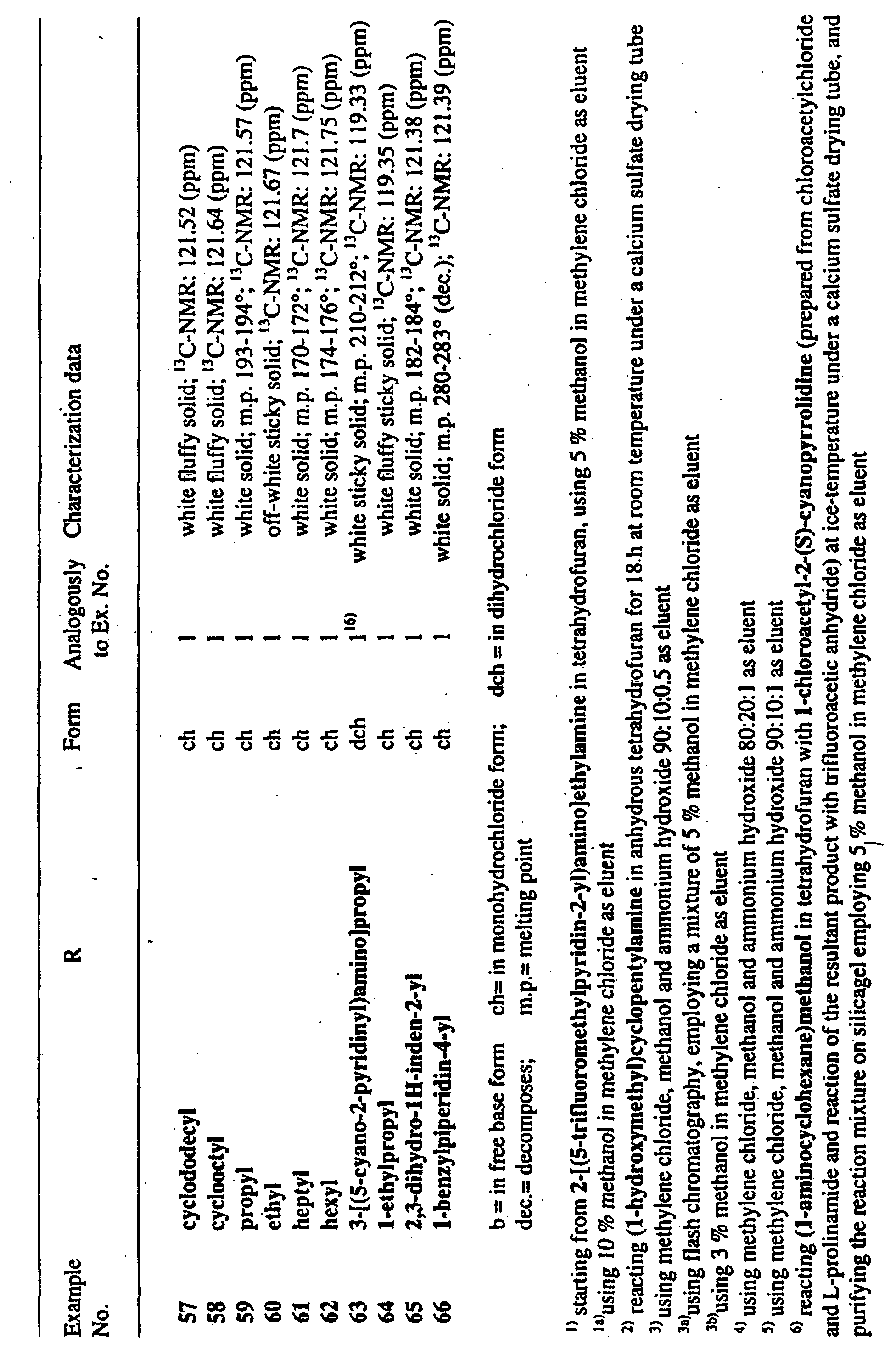

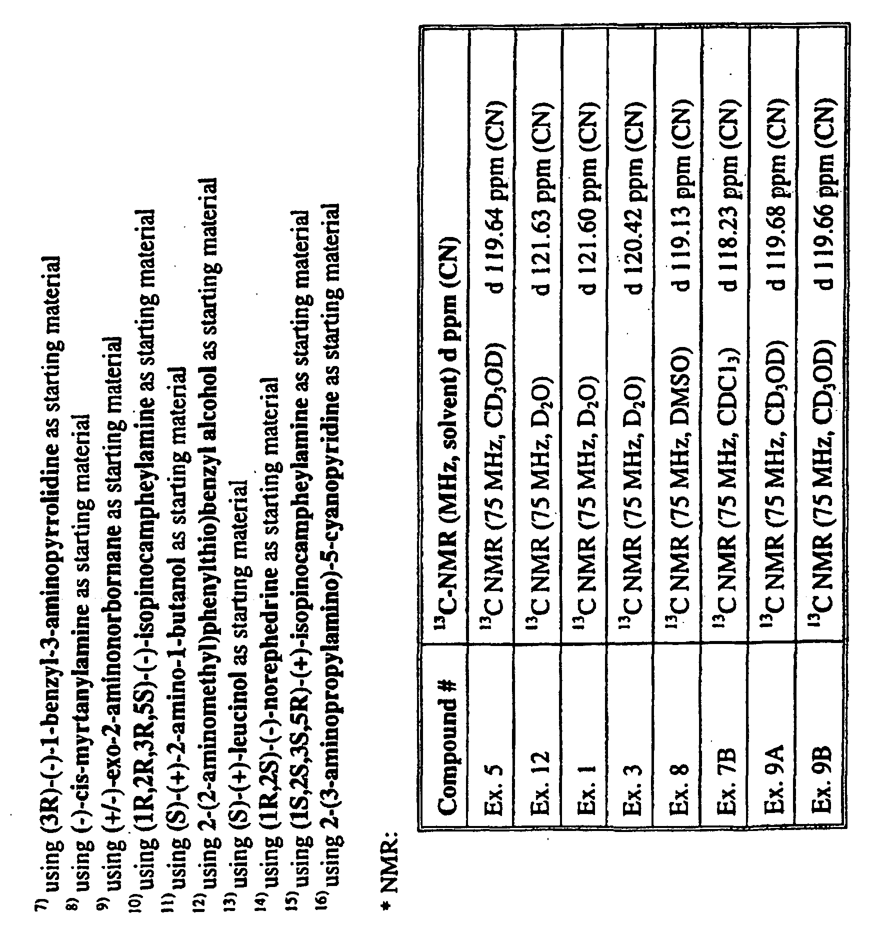

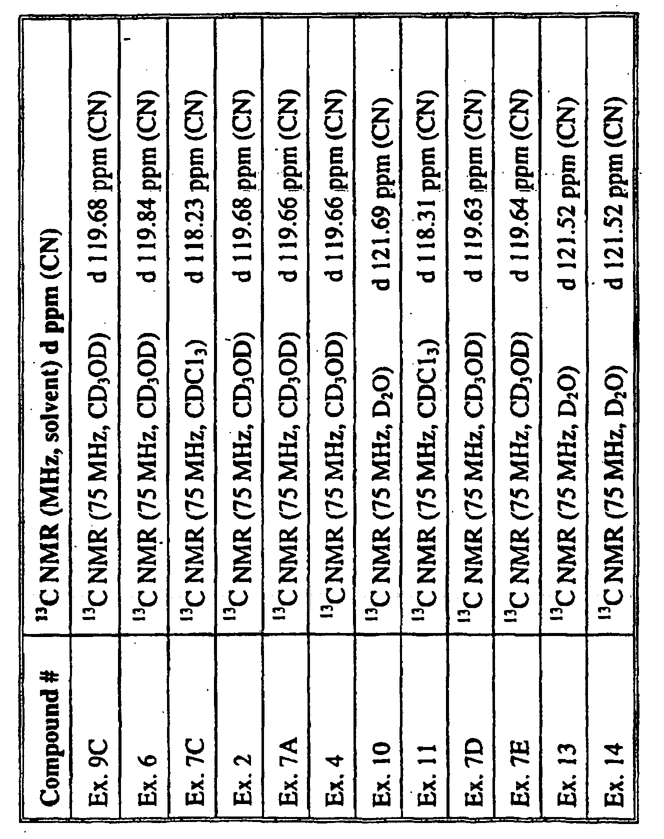

The compounds of the invention in free form or in pharmaceutically acceptable acid addition salt form, hereinafter briefly named **"the agents of the invention",** in particular, the compounds of formula I in free form or in pharmaceutically acceptable acid addition salt form, possess pharmacological activity. They are therefore indicated for use as pharmaceuticals.

In particular, they inhibit DPP-IV. This activity may be demonstrated employing the **Caco-2 DPP-IV assay,** which measures the ability of test compounds to inhibit DPP-IV activity from human colonic carcinoma cell extracts. The human colonic carcinoma cell line Caco-2 can be obtained from the American Type Culture Collection (ATCC HTB 37). Differentiation of the cells to induce DPP-IV expression is accomplished as described by Reisher et al. in Proc.Natl.Acad.Sci.USA 90 (1993) 5757-5761. Cell extract is prepared from cells solubilized in 10 mM Tris-HCl, 0.15 M NaCl, 0.04 t.i.u. (trypsin inhibitor unit) aprotinin, 0.5% non-ionic detergent P40, pH 8.0, which is centrifuged at 35 000 g for 30 min at 4°C to remove cell debris. The assay is conducted by adding 20 mg solubilized Caco-2 protein, diluted to a final volume of 125 ml in assay buffer (25 mM Tris-HCl pH 7.4, 140 mM NaCl, 10 mM KCl, 1% bovine serum albumin) to microtiter plate wells. The reaction is initiated by adding 25ml of 1 mM substrate (H-Alanine-Proline-pNA; pNA is *p*-nitroaniline). The reaction is run at room temperature for 10 minutes after which time a 19 ml volume of 25% glacial acetic acid is added to stop the reaction. Test compounds are typically added as 30 ml additions and the assay buffer volume is reduced to 95 ml. A standard curve of free *p*-nitroaniline is generated using 0-500 mM solutions of free pNA in assay buffer. The curve generated is linear and is used for interpolation of substrate consumption (catalytic activity in nmoles substrate cleaved /min). The endpoint is determined by measuring absorbance at 405 nm in a Molecular Devices UV Max microtiter plate reader. The potency of the test compounds as DPP-IV inhibitors, expressed as IC₅₀, is calculated from 8-point, dose-response curves using a 4-parameter logistic function.

In the above test, IC₅₀ values of from about 10 nM to about 900 nM are obtained with the agents of the invention, e.g. 22 nM for the agent of Example 3.

The DPP-IV inhibition may also be demonstrated by measuring the effects of test compounds on DPP-IV activity in human and rat plasma employing a modified version of the **assay described by Kubota** et al. in Clin,Exp,Immunol. 89 (1992) 192-197. Briefly, five ml of plasma are added to 96-well flat-bottom microtiter plates (Falcon), followed by the addition of 5 ml of 80 mM MgCl₂ in incubation buffer (25 mM HEPES, 140 mM NaCl,
1 % RIA-grade BSA, pH 7.8). After a 5 min incubation at room temperature, the reaction is initiated by the addition of 10 ml of incubation buffer containing 0.1 mM substrate (H-Glycine-Proline-AMC; AMC is 7-amino-4-methylcoumarin). The plates are covered with aluminum foil (or kept in the dark) and incubated at room temperature for 20 min. After the 20 min reaction, fluorescence is measured using a CytoFluor 2350 fluorimeter (Excitation 380 nm Emission 460 nm; sensitivity setting 4). Test compounds are typically added as 2 ml additions and the assay buffer volume is reduced to 13 ml. A fluorescence-concentration curve of free AMC is generated using 0-50 mM solutions of AMC in assay buffer. The curve generated is linear and is used for interpolation of substrate consumption (catalytic activity in nmoles substrate cleaved/min). As with the previous assay, the potency of the test compounds as DPP-IV inhibitors, expressed as IC₅₀, is calculated from 8-point, dose-response curves using a 4 parameter logistic function.

In the above assay, IC₅₀ values of from about 7 nM to about 2000 nM are obtained in human plasma, and of from about 3 nM to about 400 nM in rat plasma, e.g., for the agent of Example 3, of 7 nM in human and 6 nM in rat plasma, respectively.

In view of their ability to **inhibit DPP-IV,** the agents of the invention are indicated for use in **treating conditions mediated by DPP-IV.** It is expected that the compounds disclosed herein are useful in the treatment of **non-insulin-dependent diabetes mellitus, arthritis, obesity, and osteoporosis such as calcitonin-osteoporosis**. The agents of the invention improve early insulin response to an oral glucose challenge and, therefore, are particularly indicated for use in treating **non-insulin-dependent diabetes mellitus and further conditions of impaired glucose tolerance (IGT).**

The ability of the agents of the invention to improve early insulin response to an oral glucose challenge may e.g. be measured in insulin resistant rats according to the following method:

Male Sprague-Dawley rats that have been fed a high fat diet (saturated fat = 57 % calories) for 2-3 weeks are fasted for approximately 2 hours on the day of testing, divided into groups of 8-10, and dosed orally with 10 mmol/kg of the test compounds in carboxymethylcellulose (CMC). An oral glucose bolus of I g/kg is administered 30 min after the test compound directly into the stomach of the test animals. Blood samples, obtained at various timepoints from chronic jugular vein catheters are analyzed for plasma glucose and immunoreactive insulin (IRI) concentrations, and plasma DPP-IV activity. Plasma insulin levels are assayed by a double antibody radioimmunoassay (RIA) method using a specific anti-rat insulin antibody from Linco Research (St. Louis, MO, USA). The RIA has a lower limit of detection of 0.5 mU/ml with intra- and inter-assay variations of less than 5%. Data are expressed as % increase of the mean of the control animals.

It was found that upon oral administration, each of the compounds tested amplified the early insulin response which led to an improvement in glucose tolerance in the insulin resistant test animals. The following results were obtained:

| Compound | Increase of insulin response at 10 mmol/kg |
|---|---|
| Ex. 1 | 61 % |
| Ex. 3 | 66 % |
| Ex. 5 | 108 % |
| Ex. 8 | 144 % |
| Ex. 12 | 59 % |

The precise dosage of the agents of the invention to be employed for treating conditions mediated by DPP-IV inhibition depends upon several factors, including the host, the nature and the severity of the condition being treated, the mode of administration and the particular compound employed. However, in general, conditions mediated by DPP-IV inhibition are effectively treated when an agent of the invention is administered enterally, e.g. orally, or parenterally, e.g. intravenously, preferably orally, at a daily dosage of from about 0.002 mg/kg to about 5 mg/kg, preferably of from about 0.02 mg/kg to about 2.5 mg/kg body weight or, for most larger primates, a daily dosage of from about 0.1 mg to about 250 mg, preferably from about 1 mg to about 100 mg. A typical oral dosage unit is from about 0.01 mg/kg to about 0.75 mg/kg, one to three times a day. Usually, a small dose is administered initially and the dosage is gradually increased until the optimal dosage for the host under treatment is determined. The upper limit of dosage is that imposed by side effects and can be determined by trial for the host being treated.

The agents of the invention may be combined with one or more pharmaceutically acceptable carriers and, optionally, one or more other conventional pharmaceutical adjuvants and administered enterally, e.g. orally, in the form of tablets, capsules, caplets, etc., or parenterally, e.g. intravenously, in the form of sterile injectable solutions or suspensions. The enteral and parenteral compositions may be prepared by conventional means.

The agents of the invention may be formulated into enteral and parenteral pharmaceutical compositions containing an amount of the active substance that is effective for treating conditions mediated by DPP-IV, such compositions in unit dosage form and such compositions comprising a pharmaceutically acceptable carrier.

Those agents of the invention which are e.g. of formula I may be administered in enantiomerically pure (S) form (e.g. ≥ 98 %, preferably ≥ 99 % pure) or together with the other enantiomer, e.g. in racemic form. The above dosage ranges are based on the compounds of formula I (excluding the amount of R enantiomer).

The invention thus also comprises **an agent of the invention,** in particular, a compound of formula I as defined above, in free form or in pharmaceutically acceptable acid addition salt form, **for use as a pharmaceutical**. It further includes a **pharmaceutical composition** comprising an agent of the invention, in particular a compound of formula I as defined above, in free form or in pharmaceutically acceptable acid addition salt form, together with at least one pharmaceutically acceptable carrier or diluent. It further comprises the use of an agent of the invention, in particular, a compound of formula I as defined above, in free form or in pharmaceutically acceptable acid addition salt form **in the preparation of a medicament** for inhibiting DPP-IV or treating conditions mediated by DPP-IV, by a process comprising mixing an agent of the invention with a pharmaceutically acceptable carrier or diluent. It further provides a **method of inhibiting DPP-IV, or of treating conditions mediated by DPP-IV,** which comprises administering to a patient in -need of such treatment a therapeutically effective amount of a compound of the invention, in particular, of formula I in free form or in pharmaceutically acceptable acid addition salt form.

The agents of Examples 1, 3, 5, 8 and 12 are the preferred agents of the invention, particularly those of Examples 1, 3, 5 and 12, preferably in hydrochloride acid addition salt form, especially the agent of Example 3, namely 1-[2-[(5-cyanopyridin-2-yl)amino]-ethylamino]acetyl-2-cyano-(S)-pyrrolidine, preferably in dihydrochloride acid addition salt form. It has been determined that in hydrochloride form they have an IC₅₀ value in the Caco-2 DPP-IV assay of, respectively, 36, 22, 26, 8 and 279 nM, and in the modified Kubota assay above, an IC₅₀ value for, respectively, human and rat plasma DPP-IV, of 27 and 22 nM (Example 1); 7 and 6 nM (Example 3); 37 and 18 nM (Example 5); 12 and 11 nM (Example 8); and 95 and 38 nM (Example 12). It is, therefore, indicated that for the above uses the compounds of Examples 1, 3, 5, 8 and 12 may be administered to larger mammals, for example humans, by similar modes of administration at similar dosages than conventionally employed with metformin.

## Claims

1. A compound of formula I: 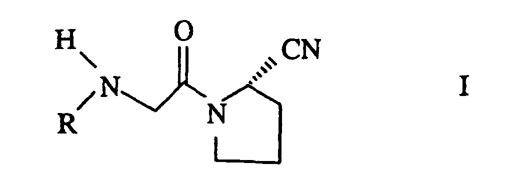 wherein **R** is:
a) **R**_{**1**}**R**_{**1a**}**N(CH**_{**2**}**)**_{**m**}**-** wherein
R₁ is a pyridinyl or pyrimidinyl moiety optionally mono- or independently disubstituted with (C₁₋₄)alkyl, (C₁₋₄)alkoxy, halogen, trifluoromethyl, cyano or nitro; or phenyl optionally mono- or independently disubstituted with (C₁₋₄)alkyl, (C₁₋₄)alkoxy or halogen;
R₁ₐ is hydrogen or (C₁₋₈)alkyl; and
m is 2 or 3;
b) **(C**_{**3-12**}**)cycloalkyl** optionally monosubstituted in the 1-position with (C₁₋₃)hydroxyalkyl;
c) **R**_{**2**}**(CH**_{**2**}**)**_{**n**}**-** wherein either
R₂ is phenyl optionally mono- or independently di- or independently trisubstituted with (C₁₋₄)alkyl, (C₁₋₄)alkoxy, halogen or phenylthio optionally monosubstituted in the phenyl ring with hydroxymethyl; or is (C₁₋₈)alkyl; a [3.1.1]bicyclic carbocyclic moiety optionally mono- or plurisubstituted with (C₁₋₈)alkyl; a pyridinyl or naphthyl moiety optionally mono- or independently disubstituted with (C₁₋₄)alkyl, (C₁₋₄)alkoxy or halogen; cyclohexene; or adamantyl; and
n is 1 to 3; or
R₂ is phenoxy optionally mono- or independently disubstituted with (C₁₋₄)alkyl, (C₁₋₄)alkoxy or halogen; and
n is 2 or 3;
d) **(R**_{**3**}**)**_{**2**}**CH(CH**_{**2**}**)**_{**2**}**-** wherein each R₃ independently is phenyl optionally mono- or independently disubstituted with (C₁₋₄)alkyl, (C₁₋₄)alkoxy or halogen;
e) **R**_{**4**}**(CH**_{**2**}**)**_{**p**}**-** wherein R₄ is 2-oxopyrrolidinyl or (C₂₋₄)alkoxy and
p is 2 to 4;
f) **isopropyl** optionally monosubstituted in 1-position with (C₁₋₃)hydroxyalkyl;
g) **R**_{**5**} wherein R₅ is: indanyl; a pyrrolidinyl or piperidinyl moiety optionally substituted with benzyl; a [2.2.1]- or [3.1.1]bicyclic carbocyclic moiety optionally mono- or plurisubstituted with (C₁₋₈)alkyl; adamantyl; or (C₁₋₈)alkyl optionally mono- or independently plurisubstituted with hydroxy, hydroxymethyl or phenyl optionally mono- or independently disubstituted with (C₁₋₄)alkyl, (C₁₋₄)alkoxy or halogen;
in free form or in acid addition salt form,

2. A compound according to claim 1 (a **compound Ip)** wherein **R** is **R**^{**p**}**,** which is:
a) R₁^{p}NH(CH₂)₂- wherein R₁^{p} is a pyridinyl or pyrimidinyl moiety optionally mono- or independently disubstituted with halogen, trifluoromethyl, cyano or nitro;
b) (C₃₋₇)cycloalkyl optionally monosubstituted in 1-position with (C₁₋₃)hydroxyalkyl;
c) R₂^{p}(CH₂)₂- wherein R₂^{p} is phenyl optionally mono- or independently di- or independently trisubstituted with halogen or (C₁₋₃)alkoxy;
d) (R₃^{p})₂CH(CH₂)₂- wherein each R₃^{p} independently is phenyl optionally monosubstituted with halogen or (C₁₋₃)alkoxy;
e) R₄(CH₂)₃- wherein R₄ is as defined above; or
f) isopropyl optionally monosubstituted in 1-position with (C₁₋₃)hydroxyalkyl;
in free form or in acid addition salt form.

3. A compound according to claim 1 (a **compound Is),** wherein R is **R**^{**s**}**,** which is:
a) R₁^{s}R₁ₐ^{s}(CH₂)ₘₛ- wherein R₁^{s} is pyridinyl optionally mono- or independently disubstituted with chlorine, trifluoromethyl, cyano or nitro; pyrimidinyl optionally monosubstituted with chlorine or trifluoromethyl; or phenyl;
R₁ₐ^{s} is hydrogen or methyl; and
ms is 2 or 3;
b) (C₃₋₁₂)cycloalkyl optionally monosubstituted in 1-position with hydroxymethyl;
c) R₂^{s}(CH₂)ₙₛ- wherein either
R₂^{s} is phenyl optionally mono- or independently di- or independently trisubstituted with halogen, alkoxy of 1 or 2 carbon atoms or phenylthio monosubstituted in the phenyl ring with hydroxymethyl; (C₁₋₆)alkyl; 6,6-dimethylbicyclo[3.1.1]hept-2-yl; pyridinyl; naphthyl; cyclohexene; or adamantyl; and ns is 1 to 3; or
R₂^{s} is phenoxy; and ns is 2;
d) (3,3-diphenyl)propyl;
e) R₄^{s}(CH₂)ₚₛ wherein R₄^{s} is 2-oxopyrrolidin-1-yl or isopropoxy and
ps is 2 or 3;
f) isopropyl optionally monosubstituted in 1-position with hydroxymethyl;
g) R₅^{s} wherein R₅^{s} is: indanyl; a pyrrolidinyl or piperidinyl moiety optionally N-substituted with benzyl; bicyclo[2.2.1]hept-2-yl; 2,6,6-trimethylbicyclo-[3.1.1]hept-3-yl; adamantyl; or (C₁₋₈)alkyl optionally mono- or independently disubstituted with hydroxy, hydroxymethyl or phenyl;
in free form or in acid addition salt form.

4. The compound according to claim 1 wherein R is 2-[(5-cyanopyridin-2-yl)amino]ethyl, i.e. 1-[2-[(5-cyanopyridin-2-yl)amino]ethylamino]acetyl-2-cyano-(S)-pyrrolidine, in free form or in acid addition salt form, especially in dihydrochloride acid addition salt form, or
a compound according to claim 2 which is of formula I wherein R is either
- 2-[(5-chloropyridin-2-yl)amino]ethyl, or
- (1-hydroxymethyl)cyclopent-1-yl, or
- 2-[(5-nitropyridin-2-yl)amino]ethyl, or
- 3-(isopropoxy)propyl,
in free form or in acid addition salt form.

5. A process for the preparation of a compound according to claim 1, which comprises reacting a compound of formula II 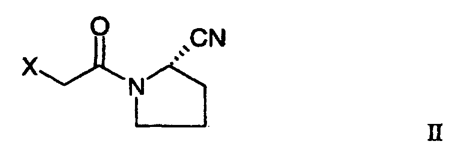 wherein X is a reactive group,
with a compound of formula III
NH₂R III
wherein R is as defined in claim 1,
and recovering the resultant compound in free or in acid addition salt form.

6. A pharmaceutical composition comprising a compound according to claim 1 in free form or in pharmaceutically acceptable acid addition salt form, together with at least one pharmaceutically acceptable carrier or diluent.

7. A compound according to claim 1 in free form or in pharmaceutically acceptable acid addition salt form, for use as a pharmaceutical.

8. Use of a compound according to claim 1 in free form or in pharmaceutically acceptable acid addition salt form in the preparation of a medicament for inhibiting DPP-IV or treating conditions mediated by DPP-IV.

9. A compound of the formula II 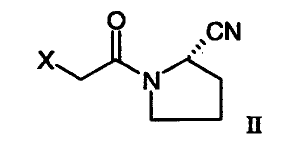 wherein X is a halogen.

10. A compound according to claim 9, wherein X is selected from the group consisting of Cl, Br, and I.

11. A compound according to claim 9, which is 1-chloroacetyl-2-cyanopyrrolidine.

## Patentansprüche

1. Verbindung der Formel 1 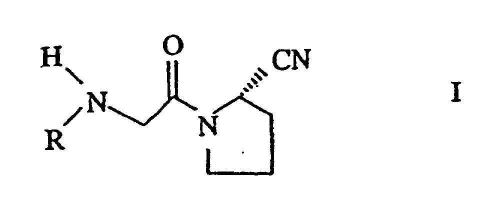 worin R steht für
a) R₁R₁ₐN(CH₂)ₘ- worin
R₁ steht für einen Pyridinyl- oder Pyrimidinylrest, der gegebenenfalls mono- oder unabhängig disubstituiert ist durch (C₁-C₄)-Alkyl, (C₁-C₄)-Alkoxy, Halogen, Trifluormethyl, Cyano oder Nitro, oder für Phenyl, das gegebenenfalls mono- oder unabhängig disubstituiert ist durch (C₁-C₄)-Alkyl, (C₁-C₄)-Alkoxy oder Halogen,
R₁ₐ für Wasserstoff oder (C₁-C₈)-Alkyl steht und
m für 2 oder 3 steht,
b) (C₃-C₁₂)-Cycloalkyl-, das in Stellung 1 gegebenenfalls monosubstituiert ist durch (C₁-C₃)-Hydroxyalkyl,
c) R₂(CH₂)ₙ-, worin entweder
R₂ steht für Phenyl, das gegebenenfalls mono- oder unabhängig di- oder unabhängig trisubstituiert ist durch (C₁-C₄)-Alkyl, (C₁-C₄)-Alkoxy, Halogen oder Phenylthio, das am Phenylring gegebenenfalls monosubstituiert ist durch Hydroxymethyl, oder für (C₁-C₈)-Alkyl, einen [3.1.1]bicyclischen carbocyclischen Rest, der gegebenenfalls mono- oder plurisubstituiert ist durch (C₁-C₈)-Alkyl, für einen Pyridinyl- oder Naphthylrest, der gegebenenfalls mono- oder unabhängig disubstituiert ist durch (C₁-C₄)-Alkyl, (C₁-C₄)-Alkoxy oder Halogen, für Cyclohexen oder Adamantyl und
n für 1 bis 3 steht, oder
R₂ für Phenoxy steht, das gegebenenfalls mono- oder unabhängig disubstituiert ist durch (C₁-C₄)-Alkyl, (C₁-C₄)-Alkoxy oder Halogen, und
n für 2 oder 3 steht,
d) (R₃)₂CH(CH₂)₂-, worin jedes R₃ unabhängig für Phenyl steht, das gegebenenfalls mono- oder unabhängig disubstituiert ist durch (C₁-C₄)-Alkyl, (C₁-C₄)-Alkoxy oder Halogen,
e) R₄(CH₂)ₚ-, worin R₄ für 2-oxopyrrolidinyl oder (C₂-C₄)-Alkoxy steht und
p für 2 bis 4 steht,
f) Isopropyl, das in Stellung 1 gegebenenfalls monosubstituiert ist durch (C₁-C₃)-Hydroxyalkyl,
g) R₅, worin R₅ steht für Indanyl, einen Pyrrolidinyl- oder Piperidinylrest, der gegebenenfalls substituiert ist durch Benzyl, für einen [2.2.1]- oder [3.1.1]bicyclischen carbocyclischen Rest, der gegebenenfalls mono- oder plurisubstituiert ist durch (C₁-C₈)-Alkyl, für Adamantyl oder (C₁-C₈)-Alkyl, das gegebenenfalls mono- oder unabhängig plurisubstituiert ist durch Hydroxy, Hydroxymethyl oder Phenyl, das gegebenenfalls mono- oder unabhängig disubstituiert ist durch (C₁-C₄)-Alkyl, (C₁-C₄)-Alkoxy oder Halogen,
in freier Form oder in Form eines Säureadditionssalzes.

2. Verbindung nach Anspruch 1 (eine Verbindung Ip), worin R für R^{p} steht, mit den Bedeutungen
a) R₁^{p}NH(CH₂)₂-, worin R₁^{p} für einen Pyridinyl- oder Pyrimidinylrest steht, der gegebenenfalls mono- oder unabhängig disubstituiert ist durch Halogen, Trifluoromethyl, Cyano oder Nitro,
b) (C₃-C₇)-Cycloalkyl, das gegebenenfalls in Stellung 1 monosubstituiert ist durch (C₁-C₃)-Hydroxyalkyl,
c) R₂^{p}(CH₂)₂-, worin R₂^{p} für Phenyl steht, das gegebenenfalls mono- oder unabhängig di- oder unabhängig trisubstituiert ist durch Halogen oder (C₁-C₃)-Alkoxy,
d) (R₃^{p})₂CH(CH₂)₂-, worin jedes R₃^{p} unabhängig für Phenyl steht, das gegebenenfalls monosubstituiert ist durch Halogen oder (C₁-C₃)-Alkoxy,
e) R₄(CH₂)₃-, worin R₄ wie oben definiert ist, oder
f) Isopropyl, das gegebenenfalls in Stellung 1 monosubstituiert ist durch (C₁-C₃)-Hydroxyalkyl,
in freier Form oder in Form eines Säureadditionssalzes.

3. Verbindung nach Anspruch 1 (eine Verbindung Is), worin R für R^{s} steht, mit den Bedeutungen
a) R₁^{s}R₁ₐ^{s}(CH₂)ₘₛ-, worin R₁^{s} steht für Pyridinyl, das gegebenenfalls mono- oder unabhängig disubstiutiert ist durch Chlor, Trifluormethyl, Cyano oder Nitro, für Pyrimidinyl, das gegebenenfalls monosubstiutiert ist durch Chlor oder Trifluormethyl, oder für Phenyl,
R₁ₐ^{s} für Wasserstoff oder Methyl steht, und
ms für 2 oder 3 steht,
b) (C₃-C₁₂)-Cycloalkyl, das gegebenenfalls in Stellung 1 monosubstituiert ist durch Hydroxymethyl,
c) R₂^{s}(CH₂)ₙₛ-, worin entweder
R₂^{s} steht für Phenyl, das gegebenenfalls mono- oder unabhängig di- oder unabhängig trisubstituiert ist durch Halogen, für Alkoxy mit 1 oder 2 Kohlenstoffatomen oder Phenylthio, das am Phenylring monosubstituiert ist durch Hydroxymethyl, für (C₁-C₆)-Alkyl, 6,6-Dimethylbicyclo[3.1.1]hept-2-yl, Pyridinyl, Naphthyl, Cyclohexen oder Adamantyl und
ns für 1 bis 3 steht, oder
R₂^{s} für Phenoxy steht und
ns für 2 steht,
d) (3,3-Diphenyl)propyl,
e) R₄^{s}(CH₂)ₚₛ-, worin R₄^{s} für 2-Oxopyrrolidin-1-yl oder Isopropoxy steht und
ps für 2 oder 3 steht,
f) Isopropyl, das in Stellung 1 gegebenenfalls monosubstituiert ist durch Hydroxymethyl,
g) R₅^{s}, worin R₅^{s} steht für Indanyl, einen Pyrrolidinyl- oder Piperidinylrest, der am N gegebenenfalls substituiert ist durch Benzyl, für Bicyclo[2.2.1]hept-2-yl, 2,6,6-Trimethylbicyclo-[3.1.1]hept-3-yl, Adamantyl oder (C₁-C₈)-Alkyl, das gegebenenfalls mono- oder unabhängig disubstituiert ist durch Hydroxy, Hydroxymethyl oder Phenyl,
in freier Form oder in Form eines Säureadditionssalzes.

4. Verbindung nach Anspruch 1, worin R für 2-[(5-Cyanopyridin-2-yl)amino]ethyl steht, nämlich 1-[2-[(5-Cyanopyridin-2-yl)amino]ethylamino]acetyl-2-cyano-(S)-pyrrolidin,
in freier Form oder in Form eines Säureadditionssalzes, insbesondere in Form des Dihydrochloridsäureadditionssalzes, oder eine
Verbindung nach Anspruch 2, die der Formel I entspricht, worin R steht für
- 2-[(5-Chlorpyridin-2-yl)amino]ethyl oder
- (1-Hydroxymethyl)cyclopent-1-yl oder
- 2-[(5-Nitropyridin-2-yl)amino]ethyl oder
- 3-(Isopropoxy)ppropyl,
in freier Form oder in Form eines Säureadditionssalzes.

5. Verfahren zur Herstellung einer Verbindung nach Anspruch 1 durch Umsetzung einer Verbindung der Formel II 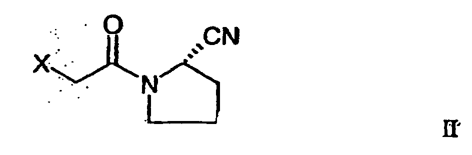 worin X eine reaktive Gruppe ist,
mit einer Verbindung der Formel III
NH₂R III
worin R wie im Anspruch 1 definiert ist,
und durch Gewinnung der erhaltenen Verbindung in freier Form oder in Form eines Säureadditionssalzes.

6. Pharmazeutische Zusammensetzung, umfassend eine Verbindung nach Anspruch 1 in freier Form oder in Form eines pharmazeutisch annehmbaren Säureadditionssalzes zusammen mit wenigstens einem pharmazeutisch annehmbaren Träger oder Verdünnungsmittel.

7. Verbindung nach Anspruch 1 in freier Form oder in Form eines pharmazeutisch annehmbaren Säureadditionssalzes zur Verwendung als Pharmazeutikum.

8. Verwendung einer Verbindung nach Anspruch 1 in freier Form oder in Form eines pharmazeutisch annehmbaren Säureadditionssalzes bei der Herstellung eines Arzneimittels zur Hemmung von DPP-IV oder zur Behandlung von durch DPP-IV mediierten Zuständen.

9. Verbindung der Formel II 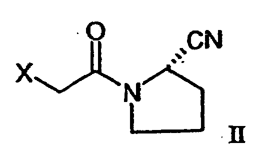 worin X für ein Halogen steht.

10. Verbindung nach Anspruch 9, worin X ausgewählt ist aus der Gruppe, die besteht aus Cl, Br und I.

11. Verbindung nach Anspruch 9, welche 1-Chloracetyl-2-cyanopyrrolidin ist.

## Revendications

1. Composé de formule 1 : 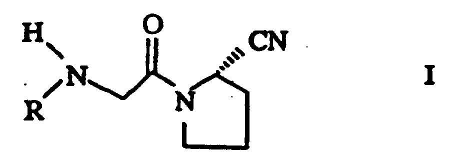 dans laquelle R représente :
a) un groupe R₁R₁ₐN(CH₂)ₘ- dans lequel
R₁ est un groupement pyridinyle ou pyrimidinyle facultativement mono- ou indépendamment disubstitué par un groupe alkyle en C₁ à C₄, alcoxy en C₁ à C₄, halogéno, trifluorométhyle, cyano ou nitro ; ou phényle facultativement mono- ou indépendamment disubstitué par un groupe alkyle en C₁ à C₄, alcoxy en C₁ à C₄ ou halogèno ;
R₁ₐ représente l'hydrogène ou un groupe alkyle en C₁ à C₈ ; et
m vaut 2 ou 3 ;
b) un groupe cycloalkyle en C₃ à C₁₂ facultativement monosubstitué en position 1 par un groupe hydroxyalkyle en C₁ à C₃ ;
c) un groupe R₂(CH₂)ₙ- dans lequel soit
R₂ est un groupe phényle facultativement mono- ou indépendamment di- ou indépendamment trisubstitué par un groupe alkyle en C₁ à C₄, alcoxy en C₁ à C₄, halogéno ou phénylthio facultativement monosubstitué dans le noyau phényle par un groupe hydroxyméthyle; ou est un groupe alkyle en C₁ à C₈ ; un groupement carbocyclique [3.1.1] bicyclique facultativement mono- ou plurisubstitué par un groupe alkyle en C₁ à C₈ ; un groupement pyridinyle ou naphtyle facultativement mono- ou indépendamment disubstitué par un groupe alkyle en C₁ à C₄, alcoxy en C₁ à C₄ ou halogèno ; cyclohexène ; ou adamantyle ; et
n a une valeur de 1 à 3 ; soit
R₂ est un groupe phénoxy facultativement mono- ou indépendamment disubstitué par un groupe alkyle en C₁ à C₄, alcoxy en C₁ à C₄ ou halogèno ; et
n a une valeur de 2 ou 3 ;
d) un groupe (R₃)₂CH(CH₂)₂- dans lequel chaque R₃ est indépendamment un groupe phényle facultativement mono- ou indépendamment disubstitué par un groupe alkyle en C₁ à C₄, alcoxy en C₁ à C₄ ou halogéno ;
e) un groupe R₄(CH₂)ₚ- dans lequel R₄ est un groupe 2-oxopyrrolidinyle ou alcoxy en C₂ à C₄, et
p a une valeur de 2 à 4 ;
f) un groupe isopropyle facultativement monosubstitué en position 1 par un groupe hydroxyalkyle en C₁ à C₃ ;
g) R₅ dans lequel R₅ représente : un groupement indanyle ; pyrrolidinyle ou pipéridinyle facultativement substitué par un groupe benzyle ; un groupement carbocyclique [2.1.1.]- ou [3.1.1] bicyclique facultativement mono- ou plurisubstitué par un groupe alkyle en C₁ à C₈ ; adamantyle ; ou alkyle en C₁ à C₈ facultativement mono- ou indépendamment plurisubstitué par un groupe hydroxy, hydroxyméthyle ou phényle facultativement mono- ou indépendamment disubstitué par un groupe alkyle en C₁ à C₄, alcoxy en C₁ à C₄ ou halogéno ;
sous forme libre ou sous forme d'un sel d'addition d'acide.

2. Composé suivant la revendication 1 (composé Ip), dans lequel R représente R^{p}, qui est :
a) un groupe R₁^{p}NH(CH₂)₂-, dans lequel R₁^{p} est un groupement pyridinyle ou pyrimidinyle facultativement mono- ou indépendamment disubstitué par un halogène, un groupe trifluorométhyle, cyano ou nitro ;
b) un groupe cycloalkyle en C₃ à C₇ facultativement monosubstitué en position 1 par un groupe hydroxyalkyle en C₁ à C₃ ;
c) un groupe R₂^{p}(CH₂)₂-, dans lequel R₂^{p} est un groupe phényle facultativement mono- ou indépendamment di- ou indépendamment trisubstitué par un halogène ou un groupe alcoxy en C₁ à C₃ ;
d) un groupe (R₃^{p})₂CH(CH₂)₂-, dans lequel chaque radical R₃^{p} représente indépendamment un groupe phényle facultativement monosubstitué par un halogène ou un groupe alcoxy en C₁ à C₃ ;
e) un groupe R₄(CH₂)₃-, dans lequel R₄ est tel que défini ci-dessus ; ou bien
f) un groupe isopropyle facultativement monosubstitué en position 1 par un groupe hydroxyalkyle en C₁ à C₃ ;
sous forme libre ou sous forme d'un sel d'addition d'acide.

3. Composé suivant la revendication 1 (composé Is), dans lequel R représente R^{s}, qui est :
a) un groupe R₁^{s}R₁ₐ^{s}(CH₂)ₘₛ-, dans lequel R₁^{s} est un groupe pyridinyle facultativement mono- ou indépendamment disubstitué par le chlore, un groupe trifluorométhyle, cyano ou nitro ; un groupe pyrimidinyle facultativement monosubstitué par le chlore ou un groupe trifluorométhyle ; ou phényle ;
R₁ₐ^{s} représente l'hydrogène ou un groupe méthyle ; et
ms a une valeur de 2 ou 3 ;
b) un groupe cycloalkyle en C₃ à C₁₂ facultativement monosubstitué en position 1 par un groupe hydroxyméthyle ;
c) un groupe R₂^{s}(CH₂)ₙₛ-, dans lequel soit
R₂^{s} est un groupe phényle facultativement mono- ou indépendamment di- ou indépendamment trisubstitué par un halogène, un groupe alcoxy de 1 ou 2 atomes de carbone ou phénylthio monosubstitué dans le noyau phényle par un groupe hydroxyméthyle ; alkyle en C₁ à C₆ ; 6,6-diméthylbicyclo[3.1.1]hept-2-yle ; pyridinyle ; naphtyle ; cyclohexène ; ou adamantyle ; et
ns a une valeur de 1 à 3 ; soit
R₂^{s} est un groupe phénoxy ; et ns vaut 2 ;
d) un groupe (3,3-diphényl)propyle ;
e) un groupe R₄^{s}(CH₂)ₚₛ, dans lequel R₄^{s} est un groupe 2-oxopyrrolidin-1-yle ou isopropoxy, et
ps a une valeur de 2 ou 3 ;
f) un groupe isopropyle facultativement monosubstitué en position 1 par un groupe hydroxyméthyle ;
g) R₅^{s}, dans lequel R₅^{s} représente : un groupe indanyle ; un groupement pyrrolidinyle ou pipéridinyle facultativement substitué en N par un groupe benzyle ; bicyclo[2.2.1]hept-2-yle ; 2,6,6-triméthylbicyclo[3.1.1]hept-3-yle ; adamantyle ; ou alkyle en C₁ à C₈ facultativement mono- ou indépendamment disubstitué par un groupe hydroxy, hydroxyméthyle ou phényle ;
sous forme libre ou sous forme d'un sel d'addition d'acide.

4. Composé suivant la revendication 1, dans lequel R est un groupe 2-[(5-cyanopyridin-2-yl)amino]éthyle, c'est-à-dire 1-[2-[(5-cyanopyridin-2-yl)amino]éthylamino]acétyl-2-cyano-(S)-pyrrolidine, sous forme libre ou sous forme d'un sel d'addition d'acide, en particulier sous forme d'un sel d'addition d'acide dichlorhydrate, ou
un composé suivant la revendication 2, qui répond à la formule I dans laquelle R représente un groupe :
- 2-[(5-chloropyridin-2-yl)amino]éthyle, ou
- (1-hydroxyméthyl)-cyclopent-1-yle, ou
- 2-[(5-nitropyridin-2-yl)amino]éthyle, ou
- 3-(isopropoxy)propyle,
sous forme libre ou sous forme d'un sel d'addition d'acide.

5. Procédé de préparation d'un composé suivant la revendication 1, qui comprend la réaction d'un composé de formule II : 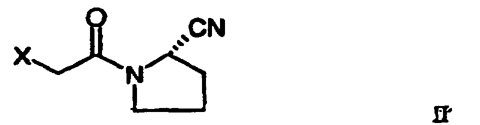 dans laquelle X est un groupe réactif,
avec un composé de formule III :
NH₂R III
dans laquelle R est tel que défini dans la revendication 1,
et la récupération du composé résultant sous forme libre ou sous forme d'un sel d'addition d'acide.

6. Composition pharmaceutique comprenant un composé suivant la revendication 1, sous forme libre ou sous forme d'un sel d'addition d'acide pharmaceutiquement acceptable, conjointement avec au moins un support ou diluant pharmaceutiquement acceptable.

7. Composé suivant la revendication 1, sous forme libre ou sous forme d'un sel d'addition d'acide pharmaceutiquement acceptable, à utiliser comme médicament.

8. Utilisation d'un composé suivant la revendication 1, sous forme libre ou sous forme d'un sel d'addition d'acide pharmaceutiquement acceptable, dans la préparation d'un médicament pour inhiber DPP-IV ou traiter des affections à médiation par DPP-IV.

9. Composé de formule II : 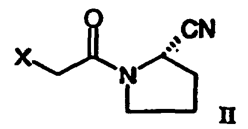 dans laquelle X est un halogène.

10. Composé suivant la revendication 9, dans lequel X est choisi d dans le groupe constitué par CI, Br et I.

11. Composé suivant la revendication 9, qui est la 1-chloroacétyl-2-cyanopyrrolidine.
